# EUROPEAN PATENT SPECIFICATION

(11) **EP 2 007 447 B1**
(45) Date of publication and mention of the grant of the patent: **30.11.2011**
(21) Application number: 07752364.5
(22) Date of filing: 05.03.2007
(51) Int. Cl.: A61L 27/58, A61L 29/14, A61L 31/14

(54) **MEDICAL DEVICES HAVING BIODEGRADABLE POLYMERIC REGIONS**
MEDIZINISCHE VORRICHTUNGEN MIT BIOLOGISCH ABBAUBAREN POLYMERREGIONEN
DISPOSITIFS MEDICAUX COMPORTANT DES REGIONS POLYMERES BIODEGRADABLES

(30) Priority: 22.03.2006 US 386273
(43) Date of publication of application: 31.12.2008
(73) Proprietor: Boston Scientific Limited, Barbados, West Indies (BB)
(72) Inventor: STECKEL, Mark, Sharon, MA 02067 (US); SIKES, Courtney, Uxbridge, MA 01569 (US)
(74) Representative: Hauck Patent- und Rechtsanwälte
(86) International application number: PCT/US2007/005654
(87) International publication number: WO 2007/111808

(56) References cited:
- EP-A- 1 555 278
- WO-A-2006/081210
- US-A1- 2005 112 172

## Description

### FIELD OF THE INVENTION

The present invention relates generally to medical devices, and more particularly to implantable or insertable medical devices which contain biodegradable polymeric regions.

### BACKGROUND OF THE INVENTION

Numerous polymer-based medical devices have been developed for implantation or insertion into the body. For example, in recent years, drug eluting coronary stents, which are commercially available from Boston Scientific Corp. (TAXUS), Johnson & Johnson (CYPHER) and others, have become the standard of care for maintaining vessel patency. These existing products are based on metallic balloon expandable stents with biostable polymer coatings, which release antiproliferative drugs at a controlled rate and total dose.

Specific examples of biostable polymers for drug eluting polymer coatings include block copolymers of polyisobutylene and polystyrene, for example, polystyrene-polyisobutylene-polystyrene triblock copolymers (SIBS copolymers), which are described in United States Patent No. 6,545,097 to Pinchuk et al., which have proven valuable in implantable and insertable medical devices for a variety of reasons, including their excellent elasticity, strength and biocompatibility. As described in Pinchuk et al., the release profile characteristics of therapeutic agents such as paclitaxel from SIBS copolymer systems demonstrate that these copolymers are effective drug delivery systems for providing therapeutic agents to sites *in vivo.*

Biodegradable polymers, on the other hand, may offer the advantage of reducing or eliminating any long term effects that may be associated with biostable polymers (e.g., foreign body effects, etc.), because they are metabolized over time.

Polymer coatings for metallic coronary stents, including biodegradable polymer coatings, should be able to withstand the strain experienced during stent deployment/expansion without cracking or an irreversible level of deformation. This requires materials with high strain-to-failure values that are typical of elastomers, but which still having adequate film strength and integrity to withstand the stresses associated with balloon expansion at high pressures (e.g., >9 atm) at 37°C in vivo.

Unfortunately, many biodegradable polymers for use in implantable and insertable medical devices either (a) form single phase systems with a glass transition temperature (Tg) above 37°C and are thus hard and substantially inelastic, leading to significant cracking and/or deformation when subjected to high strains such as those associated with stent expansion, or (b) form single phase systems with a Tg below 37°C, which, while elastic, have a low yield strength.

Block copolymers that form multiphase systems and have polymer blocks displaying Tg's both above 37°C (commonly referred to as "hard blocks") and below 37°C (commonly referred to as "soft blocks") are known, and tend to be both elastic and strong, thereby overcoming the drawbacks of the above single phase systems. However, existing biodegradable block copolymers, for example, block copolymers based on poly(butyl terephthalate), contain one or more crystalline hard blocks, leading to crystalline fragments during late stage degradation, which have been shown to result in a strong and negative reactions in vascular implants.

The above and other drawbacks are addressed by the present invention.

Document EP 1 555 278 A1 describes biodegradable multi-block copolymers comprising two different hydrolysable segments derived from two different pre-polymers A and B, wherein the pre-polymers A and B or triblock pre-polymers ABA and BAB are linked by a multi-functional chain-extender, and wherein the copolymer has a maximum glass transition temperature of 37°C under physiological (body) conditions.

Document US 2005/0112172A1 describes a biobeneficial coating composition for coating an implantable device, such as a drug eluting stent, comprising a first block copolymer and a biobeneficial polymer. The first block copolymer has a block with a Tg below about body temperature (about 37°C) and another block that has a Tg above about body temperature or has considerable crystallinity with a Tm above about body temperature.

From WO 2006/081210 A2 implantable or insertable medical devices have become known which contain polymeric release regions that release one or more therapeutic agents. The polymeric release regions, in turn, contain the following: (i) a first bonding polymer having a first polymer block and a first bonding group and (ii) a second bonding polymer having a second polymer block and a second bonding group. A therapeutic agent is disposed beneath or within the polymeric release region. The first and second polymer blocks can be the same as or different from one another, as can the first and second bonding groups.

### SUMMARY OF THE INVENTION

According to the present invention, implantable or insertable medical devices are provided, free of crystalline fragments, which contain one or more biodegradable polymeric regions. The biodegradable polymeric region comprises a biodegradable block copolymer that comprises: (a) an amorphous polymer block displaying a glass transition temperature below 37°C comprising a poly(ethylene glycol) block having an Mn in the range of from about 1000 to 2000 Daltons and (b) an amorphous biodegradable polymer block displaying a glass transition temperature above 37°C comprising amorphous poly(d,l-lactide) having an Mn in the range of from about 5000 to 20000 Daltons.

An advantage of the present invention is that medical devices (or portions thereof) may be provided which are biodegradable, strong, and elastic.

Another advantage of the present invention is that medical devices (or portions thereof) may be provided which are biodegradable, but which do not lead to crystalline fragments during the late stages of polymer degradation.

These and other aspects, embodiments and advantages of the present invention will become immediately apparent to those of ordinary skill in the art upon review of the Detailed Description and Claims to follow.

### DETAILED DESCRIPTION

According to an aspect of the present invention, implantable or insertable medical devices are provided, which consist of or contain one or more biodegradable polymeric regions. These biodegradable polymeric regions, in turn, contain one or more polymers, at least one of which is a biodegradable copolymer that includes: (a) one or more amorphous polymer blocks that display a Tg below 37°C and (b) one or more amorphous polymer blocks that display a Tg above 37°C, as defined in claim 1.

In some embodiments, the implantable or insertable medical devices contain an optional therapeutic agent, which may be disposed on, within or beneath the biodegradable polymeric regions. The at least one therapeutic agent may be released *in vivo* upon implantation or insertion of the medical device.

Biodegradable polymeric regions for use in conjunction with the present invention can correspond, for instance, to an entire device (e.g., a stent, a graft, a tissue engineering scaffold, urethral bulking beads, etc.).

On the other hand, they can also correspond, for instance, to only a portion of a medical device.

For example, the biodegradable polymeric region can be in the form of one or more fibers which are incorporated into a medical device.

In other examples, the biodegradable polymeric region can be in the form of one or more biodegradable polymeric layers that are formed over all, or only a portion of, an underlying medical device substrate, they can also be in the form of one or more biodegradable polymeric layers that are pre-formed and attached to an underlying medical device substrate, and so forth. As used herein a "layer" of a given material is a region of that material whose thickness is small compared to both its length and width. As used herein a layer need not be planar, for example, taking on the contours of an underlying substrate. Layers can be discontinuous (e.g., patterned). Terms such as "film," "layer" and "coating" may be used interchangeably herein.

Biodegradable polymeric layers in accordance with the present invention can be provided over underlying substrates at a variety of locations and in a variety of shapes. Materials for use as underlying medical device substrates include ceramic, metallic and polymeric substrates.

For example, with tubular devices such as stents (which can comprise, for example, a laser or mechanically cut tube, one or more braided, woven, or knitted filaments, etc), biodegradable polymeric layers can be provided on the luminal surfaces, on the abluminal surfaces, on the lateral surfaces between the luminal and abluminal surfaces, and so forth. Moreover, multiple biodegradable polymeric layers can be provided which contain no therapeutic agent, which contain the same therapeutic agent, or which contain, different therapeutic agents. It is therefore possible, for instance, to release the same therapeutic agent from different locations on the medical device, at the same or different rates. It is also possible to release different therapeutic agents from different locations on the medical device. For instance, it is possible to provide a tubular medical device (e.g., a vascular stent) which has a first region comprising a first therapeutic agent (e.g., an antithrombotic agent) on its inner, luminal surface and a second region comprising a second therapeutic agent (e.g., an antiproliferative agent) on its outer, abluminal surface.

Examples of medical devices to which the present invention is applicable include various implantable or insertable medical devices, for example, catheters (e.g., renal or vascular catheters such as balloon catheters and various central venous catheters), guide wires, balloons, filters (e.g., vena cava filters), stents (including coronary vascular stents, peripheral vascular stents, cerebral, urethral, ureteral, biliary, tracheal, gastrointestinal and esophageal stents), stent grafts, vascular grafts, vascular access ports, embolization devices including cerebral aneurysm filler coils (including Guglilmi detachable coils and metal coils), myocardial plugs, patches, pacemakers and pacemaker leads, left ventricular assist hearts and pumps, total artificial hearts, heart valves, vascular valves, anastomosis clips and rings, tissue bulking devices, and tissue engineering scaffolds for cartilage, bone, skin and other in vivo tissue regeneration, among others.

The medical devices of the present invention include implantable and insertable medical devices that are used for systemic treatment and those that are used for the localized treatment of any mammalian tissue or organ. Non-limiting examples are tumors; organs including the heart, coronary and peripheral vascular system (referred to overall as "the vasculature"), the urogenital system, including kidneys, bladder, urethra, ureters, prostate, vagina, uterus and ovaries, eyes, lungs, trachea, esophagus, intestines, stomach, brain, liver and pancreas, skeletal muscle, smooth muscle, breast, dermal tissue, cartilage, tooth and bone.

As used herein, "treatment" refers to the prevention of a disease or condition, the reduction or elimination of symptoms associated with a disease or condition, or the substantial or complete elimination of a disease or condition. Preferred subjects (also referred to as "patients") are vertebrate subjects, more preferably mammalian subjects and more preferably human subjects.

As used herein a "polymeric region" is region that contains one or more polymers, for example, 50 wt% or more, 75 wt% or more, 90 wt% or more, or even 95 wt% or more polymers.

A biodegradable polymeric region is one that degrades or dissolves *in vivo* upon implantation or insertion, for example, over a period of days, weeks, months or even years.

As used herein, "polymers" are molecules containing multiple (typically on the order of 5, 10, 100, 1000 or more) copies of one or more constitutional units, commonly referred to as monomers. As used herein, "homopolymers" are polymers that contain multiple copies of a single constitutional unit. "Copolymers" are polymers that contain multiple copies of at least two dissimilar constitutional units.

As previously indicated, the biodegradable polymeric regions of the invention contain one or more polymers, at least one of which is a biodegradable block copolymers that includes: (a) one or more amorphous polymer blocks that display a high Tg and (b) one or more amorphous polymer blocks that display a low Tg.

As used herein, a polymer "block" is a portion of a polymer which corresponds to a grouping of constitutional units, for example, 10, 25, 30, 100, 250, 500, 1000 or even more units. Blocks can be branched or unbranched. Blocks can contain a single type of constitutional unit (also referred to herein as "homopolymeric blocks") or multiple types of constitutional units (also referred to herein as "copolymeric blocks") which may be provided, for example, in a random, statistical, gradient, or periodic (e.g., alternating) distribution. A "chain" is a linear (unbranched) grouping of constitutional units (i.e., a linear block).

As defined herein a block displaying a "high Tg" is one displaying a Tg above 37°C (e.g., 40°C to 50°C to 75°C to 100°C to 125°C), whereas a block displaying a "low Tg" is one displaying a Tg below 37°C (e.g., -50°C to -25°C to 0°C to 25°C to 35°C).

The glass transition temperature of the polymer blocks within the biodegradable copolymer may be readily measured by differential scanning calorimetry (DSC) or other means known in the art.

Whether or not the polymer blocks are amorphous may be measured using DSC or x-ray diffraction as is known in the art.

Specific examples of amorphous, high Tg biodegradable polymer blocks include suitable amorphous homopolymer blocks or copolymer blocks which comprise one or more poly(hydroxy acids), such as amorphous poly(d,l-lactide) blocks.

Specific examples of amorphous, low Tg biodegradable polymer blocks include suitable amorphous homopolymer blocks or copolymer blocks which comprise one or more alkylene oxides, such as poly(ethylene glycol) blocks.

The biodegradable block copolymers for use in the biodegradable polymeric regions of the invention may take on a number of configurations, which may be selected, for example, from cyclic, linear and branched configurations. Branched configurations include star-shaped configurations (e.g., configurations in which three or more chains emanate from a single branch point), comb configurations (e.g., configurations having a main chain and a plurality of side chains), dendritic configurations (e.g., arborescent and hyperbranched polymers), and so forth.

A few examples are set forth below for copolymers that contain linear, amorphous, high Tg biodegradable polymer blocks "H" and linear, amorphous, low Tg biodegradable polymer blocks "L". As noted above, these blocks can be homopolymer blocks or copolymer blocks (for instance, copolymer blocks containing random, periodically repeating, etc. constitutional units). Examples include biodegradable block copolymers having the following structures: (a) HLₙ or LHₙ, where n is an integer, for example, HL (diblock), HLH or LHL (triblock copolymers), HL₃ or LH₃ (three-arm, star-shaped copolymers), and so forth. Other examples include alternating configurations such as (HL)ₙ, L(HL)ₙ, (LH)ₙ or H(LH)ₙ. Note that it is common to disregard the presence of small entities X (e.g., seed molecules, linking groups, etc.) in describing block copolymers, for example, with LH-X-HL being commonly designated as a triblock copolymer LHL.

An exemplary embodiment utilizes an HLH triblock copolymer where the "H" blocks comprise amorphous poly(*d,l*-lactide) having a Tg of about 65°C and the "L" block comprises polyethylene glycol with a Tg of about -20°C. The Mn of the H blocks may range from about 5000 to 20000 daltons each, whereas the Mn of the L blocks may range from about 1000 to 2000 daltons. At poly(ethylene glycol) block length above about 2000 daltons, crystallization during processing may become likely, which is antithetical to the present invention.

Triblock copolymers such as those above may be polymerized using polymerization methods such as those taught in biodegradable polymer art, such as ring opening polymerization techniques. Alternatively, mono-end-functionalized poly(d,l-lactide) may be covalently coupled with di-end-functionalized poly(ethylene glycol) using an appropriate coupling reaction to form the triblocks.

Other examples include copolymers that contain a linear, amorphous, high Tg biodegradable polymer block as a main chain and numerous linear, amorphous, low Tg biodegradable polymer blocks as side chains (i.e., a block copolymer having a comb architecture), or vice versa.

Where one or more therapeutic agents are included in the medical devices of the invention, they can be incorporated in a number of ways, for example, by providing the therapeutic agent(s) at the surfaces of the biodegradable polymeric regions, by providing the therapeutic agent(s) within the biodegradable polymeric regions (in which case the polymeric regions may be referred to as "carrier regions"), by providing the therapeutic agent(s) beneath the biodegradable polymeric regions (in which case the polymeric regions may be referred to as "barrier regions"), and so forth.

For example, in some embodiments, a biodegradable carrier region may constitute the entirety of the medical device. In other embodiments, a biodegradable carrier region may be provided which corresponds to only a portion of the device, for example, disposed over all or a portion of a medical device substrate in the form of a layer. In some embodiments, a biodegradable barrier region may be provided that surrounds a source of therapeutic agent. In other embodiments, a biodegradable barrier region may be provided that over a source of therapeutic agent, which is in turn disposed over all or a portion of a medical device substrate.

A wide range of therapeutic agent loadings may be used in conjunction with the medical devices of the present invention, with the therapeutically effective amount being readily determined by those of ordinary skill in the art and ultimately depending, for example, upon the condition to be treated, the age, sex and condition of the patient, the nature of the therapeutic agent, the nature of the medical device, the nature of the biodegradable polymeric region(s), and so forth.

Therapeutic agent release from carrier regions and barrier regions may arise from various phenomena, including release due to diffusion through the regions, release due to biodegradation of the regions, and so forth. For example, essentially all of the therapeutic agent may be released by diffusion prior to biodegradation of the polymeric region (e.g., due to substantial agent diffusivity within the polymeric region), essentially all of the therapeutic agent may be released as the polymeric region degrades (e.g., due to minimal agent diffusivity of the agent within the polymeric region), or a combination of both (e.g., where some of the agent diffuses from the device and some of the agent is trapped within or beneath the polymeric region, only to be released in conjunction with polymer degradation).

The release profiles associated with the biodegradable polymeric regions of the present invention may be modified in a number of ways, including changing the composition, molecular weight and/or architecture of the polymer blocks that form the biodegradable polymer regions.

The release profiles associated with the biodegradable polymeric regions of the present invention may also be modified by changing the size of the biodegradable polymeric regions (e.g., where layers are employed, by changing the surface area and/or thickness of the layers), changing the number of the biodegradable polymeric regions, stacking biodegradable polymeric regions on one another, and so forth.

Multiple biodegradable polymeric regions can be employed to modify the release profile, for example, (a) a barrier layer can be positioned over a carrier layer, (b) multiple carrier layers, either of the same or different content (e.g., different polymer and/or therapeutic agent content) can be stacked on top of one another, either with or without intervening barrier layers, and (c) multiple carrier layers of differing compositions can be positioned laterally to one another, among many other possibilities.

Therapeutic agents may be used singly or in combination in the medical devices of the present invention. "Drugs," "therapeutic agents," "pharmaceutically active agents," "pharmaceutically active materials," and other related terms may be used interchangeably herein. These terms include genetic therapeutic agents, non-genetic therapeutic agents and cells.

Exemplary non-genetic therapeutic agents for use in connection with the present invention include: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/ antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (l) antimicrobial agents such as triclosan, cephalosporins, aminoglycosides and nitrofurantoin; (m) cytotoxic agents, cytostatic agents and cell proliferation affectors; (n) vasodilating agents; (o)agents that interfere with endogenous vasoactive mechanisms; (p) inhibitors of leukocyte recruitment, such as monoclonal antibodies; (q) cytokines; (r) hormones; (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin; (t) beta-blockers, (u) bARKct inhibitors, (v) phospholamban inhibitors, (w) Serca 2 gene/protein, (x) immune response modifiers including aminoquizolines, for instance, imidazoquinolines such as resiquimod and imiquimod, (y) human apolioproteins (e.g., AI, AII, AIII, AIV, AV, etc.).

Preferred non-genetic therapeutic agents include paclitaxel, (including particulate forms thereof, for instance, protein-bound paclitaxel particles such as albumin-bound paclitaxel nanoparticles, e.g., ABRAXANE), sirolimus, everolimus, tacrolimus, Epo D, dexamethasone, estradiol, halofuginone, cilostazole, geldanamycin, ABT-578 (Abbott Laboratories), trapidil, liprostin, Actinomcin D, Resten=NG, Ap-17, abciximab, clopidogrel, Ridogrel, beta-blockers, bARKct inhibitors, phospholamban inhibitors, Serca 2 gene/protein, imiquimod, human apolioproteins (e.g., AI-AV), growth factors (e.g., VEGF-2), as well a derivatives of the forgoing, among others.

Exemplary genetic therapeutic agents for use in connection with the present invention include anti-sense DNA and RNA as well as DNA coding for the various proteins (as well as the proteins themselves): (a) anti-sense RNA, (b) tRNA or rRNA to replace defective or deficient endogenous molecules, (c) angiogenic and other factors including growth factors such as acidic and basic fibroblast growth factors, vascular endothelial growth factor, endothelial mitogenic growth factors, epidermal growth factor, transforming growth factor α and β, platelet-derived endothelial growth factor, platelet-derived growth factor, tumor necrosis factor a, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as - adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers PVP, SP1017 (SUPRATEK), lipids such as cationic lipids, liposomes, lipoplexes, nanoparticles, or microparticles, with and without targeting sequences such as the protein transduction domain (PTD).

Cells for use in connection with the present invention include cells of human origin (autologous or allogeneic), including whole bone marrow, bone marrow derived mono-nuclear cells, progenitor cells (e.g., endothelial progenitor cells), stem cells (e.g., mesenchymal, hematopoietic, neuronal), pluripotent stem cells, fibroblasts, myoblasts, satellite cells, pericytes, cardiomyocytes, skeletal myocytes or macrophage, or from an animal, bacterial or fungal source (xenogeneic), which can be genetically engineered, if desired, to deliver proteins of interest.

Numerous therapeutic agents, not necessarily exclusive of those listed above, have been identified as candidates for vascular treatment regimens, for example, as agents targeting restenosis. Such agents are useful for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) Angiotensin Converting Enzyme (ACE) inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (l) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/an ti oxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-[beta] pathway agents such as polyanionic agents (heparin, fucoidin), decorin, and TGF-[beta] antibodies, EGF pathway agents such as EGF antibodies, receptor antagonists and chimeric fusion proteins, TNF-[alpha] pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate , nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalam[iota]ne), rapamycin, cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

Numerous additional therapeutic agents useful for the practice of the present invention are also disclosed in U.S. Patent No. 5,733,925 assigned to NeoRx Corporation.

Various techniques are available for forming medical devices in accordance with the present invention.

For example, in embodiments where the polymer(s) making up the biodegradable polymeric regions have thermoplastic characteristics, a variety of standard thermoplastic processing techniques can be used to form biodegradable polymeric regions of various shapes, including compression molding, injection molding, blow molding, spinning, vacuum forming and calendaring, as well as extrusion into sheets, fibers, rods, tubes and other cross-sectional profiles of various lengths. Using these and other thermoplastic processing techniques, entire articles or portions thereof can be made.

In other embodiments, solvent-based techniques are used to form biodegradable polymeric regions of various shapes. Using these techniques, biodegradable polymeric regions can be formed by providing a solution that contains a solvent and polymer(s) of choice. The solvent that is ultimately selected will contain one or more solvent species, which are generally selected based on their ability to dissolve the polymer(s) making up the biodegradable polymeric region, as well as other factors, including drying rate, surface tension, etc. Generally, several solvents will be tested to see which provides biodegradable polymeric regions having the best characteristics. Solvent-based techniques include, but are not limited to, solvent casting techniques, spin coating techniques, web coating techniques, solvent spraying techniques, dipping techniques, techniques involving coating via mechanical suspension including air suspension, ink jet techniques, electrostatic techniques, and combinations of these processes.

In various embodiments of the invention, a solution (where solvent-based processing is employed) or melt (where thermoplastic processing is employed) is applied to a substrate to form a desired region. For example, the substrate can correspond to all or a portion of a medical article surface to which a layer is applied. The substrate can also be, for example, a template, such as a mold, from which the region is removed after solidification. In other embodiments, for example, extrusion and co-extrusion techniques, one or more polymeric regions may be formed without the aid of a substrate.

So long as the drug and/or any other optional agents are stable under processing conditions, then they may be provided within the solution or melt and processed to form carrier regions. Alternatively, the drug and/or other optional agents may be introduced subsequent to the formation of the biodegradable polymeric region in some instances. For instance, in some embodiments, the drug and/or any optional agents may be dissolved or dispersed within a solvent, and the resulting solution contacted with a previously formed biodegradable polymeric region (e.g., using one or more of the application techniques described above, such as dipping, spraying, etc.).

Biodegradable polymeric regions are provided over therapeutic-agent- containing regions in some embodiments of the invention (e.g., where the biodegradable polymeric region acts as a barrier region). In these embodiments, for example, a biodegradable polymeric region can be formed over a therapeutic-agent- containing region, for instance, one formed using one of the solvent based or thermoplastic techniques described above. Alternatively, a previously formed biodegradable polymeric region may be adhered over a therapeutic-agent-containing region.

## Claims

1. An implantable or insertable medical device free of crystalline fragments and comprising a biodegradable polymeric region, said biodegradable polymeric region comprising a biodegradable block copolymer that comprises: (a) an amorphous polymer block displaying a glass transition temperature below 37°C comprising a polyethylene glycol) block having an Mn in the range of from 1000 to 2000 Daltons and (b) an amorphous biodegradable polymer block displaying a glass transition temperature above 37°C comprising amorphous poly(d, 1-lactide) having an Mn in the range of from 5000 to 20000 Daltons.

2. The implantable or insertable medical device of claim 1, wherein said medical device is selected from a stent, a stent graft, a vascular graft, a guide wire, a balloon, a vena cava filter, a cerebral aneurysm filler coil, a myocardial plug, a heart valve, a vascular valve, and a tissue engineering scaffold,

3. The implantable or insertable medical device of claim 1, wherein said biodegradable polymer comprises a plurality of said amorphous polymer blocks displaying a glass transition temperature between -50°C and 0°C or between 50°C and 100°C.

4. The implantable or insertable medical device of claim 1, wherein said biodegradable copolymer is a triblock copolymer that comprises: (a) a single amorphous polymer block displaying a glass transition temperature below 37°C and (b) two amorphous polymer blocks displaying a glass transition temperature above 37°C.

5. The implantable or insertable medical device of claim 1, comprising a plurality of said biodegradable polymeric regions.

6. The implantable or insertable medical device of cl aim 1, wherein said biodegradable polymeric region is in the form of a fiber.

7. The implantable or insertable medical device of claim 1, wherein said biodegradable polymeric region is in the form of a biodegradable polymeric layer disposed over a medical device substrate.

8. The implantable or insertable medical device of claim 1, further comprising a therapeutic agent disposed beneath or within said biodegradable polymeric region.

9. The implantable or insertable medical device of claim 1, wherein said biodegradable polymeric region is in the form of a biodegradable polymeric layer, and wherein said polymeric layer is disposed over a region comprising a therapeutic agent.

10. The implantable or insertable medical device of claim 1, wherein said biodegradable polymeric region is in the form of a biodegradable polymeric layer, and wherein said polymeric layer comprises a therapeutic agent.

11. The implantable or insertable medical device of claim 1, wherein said biodegradable polymeric region is obtainable by evaporation of solvent from a solution or dispersion that comprises said solvent and said biodegradable copolymer.

12. The implantable or insertable medical device of claim 1, wherein said biodegradable polymeric region is obtainable by cooling a melt that comprises said biodegradable copolymer.

## Patentansprüche

1. Implantierbare oder einführbare medizinische Vorrichtung, frei von kristallinen Fragmenten und umfassend eine biologisch abbaubare Polymerregion, wobei die biologisch abbaubare Polymerregion ein biologisch abbaubares Block-Copolymer umfasst, das umfasst: (a) einen amorphen Polymer-Block, der eine Glasübergangstemperatur unter 37 °C zeigt, umfassend einen Polyethylenglykol-Block, der ein Mn in der Größenordnung von 1000 bis 2000 Dalton aufweist, und (b) einen amorphen, biologisch abbaubaren Polymer-Block, der eine Glasübergangstemperatur über 37 °C zeigt, umfassend amorphes Poly(D, L-Laktid) das ein Mn in der Größenordnung von 5000 bis 20.000 Dalton aufweist.

2. Implantierbare und einführbare medizinische Vorrichtung nach Anspruch 1, wobei die medizinische Vorrichtung aus einem Stent, einer Stentprothese, einer Gefäßprothese, einem Führungsdraht, einem Ballon, einem Kavafilter, einer Hirnaneurysma-Auffüllspirale, einem Herzmuskelstopfen, einer Herzklappe, einer Gefäßklappe, und einem Gewebeentwicklungsgerüst ausgewählt wird.

3. Implantierbare oder einführbare medizinische Vorrichtung nach Anspruch 1, wobei das biologisch abbaubare Polymer eine Mehrheit amorpher PolymerBlöcke umfasst, die eine Glasübergangstemperatur zwischen -50 °C und 0 °C oder zwischen 50 °C und 100 °C zeigen.

4. Implantierbare oder einführbare medizinische Vorrichtung nach Anspruch 1, wobei das biologisch abbaubare Copolymer ein Triblockcopolymer ist, das umfasst: (a) einen einzigen amorphen Polymer-Block, der eine Glasübergangstemperatur unter 37 °C zeigt, und (b) zwei amorphe PolymerBlöcke, die eine Glasübergangstemperatur über 37 °C zeigen.

5. Implantierbare oder einführbare medizinische Vorrichtung nach Anspruch 1, umfassend eine Mehrheit der biologisch abbaubaren Polymerregionen.

6. Implantierbare oder einführbare medizinische Vorrichtung nach Anspruch 1, wobei die biologisch abbaubare Polymerregion die Form einer Faser hat.

7. Implantierbare oder einführbare medizinische Vorrichtung nach Anspruch 1, wobei die biologisch abbaubare Polymerregion die Form einer biologisch abbaubaren Polymerschicht hat, die über das Substrat einer medizinischen Vorrichtung verteilt angeordnet ist.

8. Implantierbare oder einführbare medizinische Vorrichtung nach Anspruch 1, weiterhin umfassend ein therapeutisches Mittel, das unter oder in der biologisch abbaubaren Polymerregion angeordnet ist.

9. Implantierbare oder einführbare medizinische Vorrichtung nach Anspruch 1, wobei die biologisch abbaubare Polymerregion die Form einer biologisch abbaubaren Polymerschicht hat und wobei die Polymerschicht verteilt über eine Region angeordnet ist, die ein therapeutisches Mittel umfasst.

10. Implantierbare oder einführbare medizinische Vorrichtung nach Anspruch 1, wobei die biologisch abbaubare Polymerregion die Form einer biologisch abbaubaren Polymerschicht hat und wobei die Polymerschicht ein therapeutisches Mittel umfasst.

11. Implantierbare oder einführbare medizinische Vorrichtung nach Anspruch 1, wobei die biologisch abbaubare Polymerregion durch Verdampfung eines Lösungsmittels aus einer Lösung oder Dispersion erhalten wird, die das Lösungsmittel und das biologisch abbaubare Copolymer umfasst.

12. Implantierbare oder einführbare medizinische Vorrichtung nach Anspruch 1, wobei die biologisch abbaubare Polymerregion durch Kühlung einer Schmelze erhalten wird, die das biologisch abbaubare Copolymer umfasst.

## Revendications

1. Dispositif médical implantable ou insérable exempt de fragments cristallins et comprenant une région polymère biodégradable, ladite région polymère biodégradable comprenant un copolymère séquencé biodégradable qui comprend : (a) un bloc polymère amorphe présentant une température de transition vitreuse inférieure à 37°C comprenant un bloc de poly(éthylèneglycol) ayant un Mn dans la plage allant de 1000 à 2000 Dalton et (b) un bloc polymère biodégradable amorphe présentant une température de transition vitreuse supérieure à 37°C comprenant du poly(d,l-lactide) amorphe ayant un Mn dans la plage allant de 5000 à 20000 Dalton.

2. Dispositif médical implantable ou insérable selon la revendication 1, dans lequel ledit dispositif médical est sélectionné parmi une endoprothèse vasculaire, une greffe d'endoprothèse vasculaire, un fil guide, un ballon, un filtre en parapluie, une spiral de filtre d'anévrysme cérébral, un bouchon myocardique, une valve cardiaque, une valve vasculaire et un échafaudage d'ingénierie tissulaire.

3. Dispositif médical implantable ou insérable selon la revendication 1, dans lequel ledit polymère biodégradable comprend une pluralité desdits blocs polymères amorphes présentant une température de transition vitreuse comprise entre -50°C et 0°C ou entre 50°C et 100°C.

4. Dispositif médical implantable ou insérable selon la revendication 1, dans lequel ledit copolymère biodégradable est un copolymère tribloc qui comprend : (a) un seul bloc polymère amorphe présentant une température de transition vitreuse inférieure à 37°C et (b) deux blocs polymères amorphes présentant une température de transition vitreuse supérieure à 37°C.

5. Dispositif médical implantable ou insérable selon la revendication 1, comprenant une pluralité desdites régions polymères biodégradables.

6. Dispositif médical implantable ou insérable selon la revendication 1, dans lequel ladite région polymère biodégradable est sous la forme d'une fibre.

7. Dispositif médical implantable ou insérable selon la revendication 1, dans lequel ladite région polymère biodégradable est sous la forme d'une couche polymère biodégradable disposée sur un substrat du dispositif médical.

8. Dispositif médical implantable ou insérable selon la revendication 1, comprenant en outre un agent thérapeutique disposé en dessous ou au sein de ladite région polymère biodégradable.

9. Dispositif médical implantable ou insérable selon la revendication 1, dans lequel ladite région polymère biodégradable est sous la forme d'une couche polymère biodégradable, et dans lequel ladite couche polymère est disposée sur une région comprenant un agent thérapeutique.

10. Dispositif médical implantable ou insérable selon la revendication 1, dans lequel ladite région polymère biodégradable est sous la forme d'une couche polymère biodégradable, et dans lequel ladite couche polymère comprend un agent thérapeutique.

11. Dispositif médical implantable ou insérable selon la revendication 1, dans lequel ladite région polymère biodégradable peut être obtenue par évaporation de solvant à partir d'une solution ou dispersion qui comprend ledit solvant et ledit copolymère biodégradable.

12. Dispositif médical implantable ou insérable selon la revendication 1, dans lequel ladite région polymère biodégradable peut être obtenue par refroidissement d'une masse fondue qui comprend ledit copolymère biodégradable.
